Europäisches Patentamt

European Patent Office

Office européen des brevets

(11) Publication number: **0 343 635 B1**

# EUROPEAN PATENT SPECIFICATION

(45) Date of publication of patent specification: **24.08.94**    (51) Int. Cl.⁵: **C12N 5/02**

(21) Application number: **89109395.7**

(22) Date of filing: **24.05.89**

(54) **Process for continuously culturing adherent animal cells.**

(30) Priority: **25.05.88 JP 125664/88**
       **19.08.88 JP 204733/88**

(43) Date of publication of application:
**29.11.89 Bulletin 89/48**

(45) Publication of the grant of the patent:
**24.08.94 Bulletin 94/34**

(84) Designated Contracting States:
**CH DE FR GB LI**

(56) References cited:
**EP-A- 0 229 289
WO-A-84/03710
WO-A-88/00967
US-A- 4 673 649
US-A- 4 767 704**

**PROCEEDINGS OF THE NATIONAL ACADEMY
OF SCIENCES, vol. 53, 1965,pages 288-293;
R.G. HAM, "Clonal growth of mammalian
cells in a chemically defined, synthetic me-
dium"**

(73) Proprietor: **TEIJIN LIMITED**
**6-7, Minamihonmachi 1-chome
Chuo-ku
Osaka-shi Osaka 541 (JP)**

(72) Inventor: **Takazawa, Yoshiharu**
**Esupowaru-taira No. 203
6-2-2 Minamidaira
Hino-shi Tokyo (JP)**
Inventor: **Tokashiki, Michiyuki**
**1-13-8, Uchikoshi-cho
Hachioji-shi Tokyo (JP)**

(74) Representative: **Kraus, Walter, Dr. et al**
**Patentanwälte Kraus, Weisert & Partner
Thomas-Wimmer-Ring 15
D-80539 München (DE)**

THE JOURNAL OF INVESTIGATIVE DERMA-TOLOGY, vol. 81, no. 1, 1983, pages 33s-40s, The Williams & Wilkins Co., US; S.T. BOYCE et al.: "Calcium- regulated differentiation of normal human epidermal keratinocytes in chemically defined clonal culture and serum-free serial culture"

BIOCHEMICAL METHODS IN CELL CULTURE AND VIROLOGY, 1977, pages 45-89, Dowden, Hutchinson & Ross, Stroudsburg, Penn., US; R.J. KUCHLER: "Milieu for maintaining and growing animal cells In Vitro"

R. IAN FRESHNEY: "Culture of animal cells", A Manual of Basic Technique, second Edition, 1987, pages 74-78, 309-312, Alan R. Liss, Inc., New York, US

**Description**

This invention relates to a process for continuously culturing adherent animal cells, and more specifically, to a process for continuously culturing adherent animal cells in suspension.

Culturing of cells in large quantities on an industrial scale is a technique which is important in the production of various hormones, enzymes, lymphokines, nucleic acids, antibiotics and other useful biologically active substances. In many cases, cells producing these biologically active useful substances, particularly transformed cells having inserted DNA so as to permit secretion of the desired substances are adherent, and it is an industrially important problem to develop a technique of efficiently culturing these cells.

Some methods of culturing large amounts of adherent cells and devices therefor have previously been proposed. Many of them, however, are directed to the growth of the cells adhering to the surface of a solid carrier, and give rise to problems in regard to scale-up, operability and stability in long-term continuous operation. For example, the microcarrier culturing method developed by Van Wezel is excellent and involves culturing cells on microcarriers whereby they can be cultured in suspension in a tank [see Growth of Cell Strains and Primary Cells on Microcarriers in Homogeneous Culture, Nature 216, 64-65 (1967)]. However, according to the culturing method of Van Wezel et al., the culture area depends upon the area of the microcarrier, and when the culturing is continued for a long period of time, the cells are gradually come off from the microcarriers.

U. S. Patent No. 4,059,485 describes an attempt to culture adherent cells in suspension in a serum-containing medium. This method, however, has the disadvantage that as the culture is continued, the adherent cells aggregate to form large masses, and the cells in the large masses necrotize.

To the best of the knowledge of the present inventors, there has been no industrial technique of culturing adherent animal cells in suspension by themselves continuously over long periods of time without forming large aggregated cell clumps.

R.I. Freshney, Culture of Animal Cells, A Manual of Basic Technique, second edition, 1987, Chapter 7, pages 54-84 and Chapter 23, pages 309-312 discloses general methods for culturing animal cells. It is described that, in order to reduce aggregation and attachment of adherent cells in suspension, a variant of Eagle's minimum essential medium should be employed which is deficient or reduced in $Ca^{2+}$ ions.

US-A-4,673,649 discloses a process for culturing human keratinocyte cells in monolayer culture in serum-free medium at very low concentrations of calcium ion (0.01 - 0.1 mM) in order to avoid stratification and terminal differentiation of cells and to improve cellular multiplication.

It is an object of this invention to provide a process by which adherent animal cells can be cultured in suspension without using solid carriers such as microcarriers.

Another object of this invention is to provide an industrial process by which adherent animal cells can be cultured in suspension continuously over long periods of time.

Still another object of this invention is to provide an industrial process by which adherent animal cells can be cultured continuously over long periods of time while maintaining the cells themselves or relatively small aggregated particles in suspension.

Yet another object of this invention is to provide a process by which adherent animal cells can be cultured in suspension in a very high density.

A further object of this invention is to provide a process by which adherent animal cells can be cultured in suspension by using a serum-free culture medium.

A still further object of this invention is to provide a process by which adherent animal cells which secrete a useful biologically active substance are cultured in suspension, the culture broth is taken out from the culture tank, and the useful biologically active substance is recovered continuously and stably from the culture broth.

In order to achieve the above objects of the invention, the present inventors have undertaken investigations on a process by which adherent animal cells can be cultured in suspension without using solid carriers such as microcarriers. These investigations led to the finding that the concentration of a calcium ion in the medium has closely to do with the aggregation of the cells into masses. In serum-containing media or certain serum-free media generally used in the culturing of animal cells, a calcium ion exists in the form of calcium chloride, calcium pantothenate or another calcium salt, usually in a concentration of 0.3 mM, in many cases more than 0.5 mM.

When adherent animal cells are cultured in suspension in a medium with an ordinary calcium concentration, the cells gradually adhere to one another and clump or aggregate and finally become large masses.

3

When the number of cells per mass is at least 100, particularly more than 300, oxygen and nutrients do not sufficiently reach cells existing in the interior of the masses, and they lose the proliferating ability and the ability to secrete the useful biologically active substance, and in many cases die away.

It has been found unexpectedly that if the calcium ion concentration in the medium under the culturing conditions is decreased, particularly to less than 0.3 mM, preferably less than 0.25 mM, the adherent animal cells even in a high density do not become large masses and can be cultured for long periods of time in suspension by themselves or as relatively small particles.

It has also been found that if the calcium ion concentration in the medium is decreased below 0.02 mM, particularly below 0.002 mM, the growth or proliferation of the cells becomes difficult, or their ability to secrete the useful biologically active substance is abruptly decreased, but that by maintaining the calcium ion concentration within a specific range, the adherent animal cells can be grown in suspension for long periods of time, and their ability to secrete the useful biologically active substances is not adversely affected.

According to the present invention, there is provided a process for continuously culturing adherent animal cells selected from 293 strain derived from human fetal kidney cells and transformants thereof which are capable of secreting a useful biologically active substance in a serum-free medium, characterized in that

(1) a fresh medium is fed into a culture vessel, and a spent medium is withdrawn from the vessel,

(2) the adherent animal cells in the serum-free medium in the vessel are maintained in suspension,

(3) the adherent animal cells in suspension are caused to exist at a density of at least $3 \times 10^6$ cells/ml, and

(4) the concentration of a calcium ion in the medium under the culturing conditions is maintained at 0.002 mM to 0.3 mM.

Some pieces of information are available from the prior literature on the effect of a calcium ion upon the cultivation of animal cells. For example, the following references may be cited, and will be discussed at some length.

(a) Science, vol. 130, 432-437 (1959)

This is a paper entitled "Amino Acid Metabolism in Mammalian Cell Cultures". Table 1 at page 433 shows a minimum essential medium for cultivation of mammalian cells in either monolayer or suspension. It is described that the concentration of the calcium ion ($Ca^{++}$) in this essential medium is 1.8 mM or 0 (see the row for $CaCl_2$ in the column of component).

As an explanation of Table 1, it is stated: "In using this medium for the growth of cells in suspension, $Ca^{++}$ should be omitted or greatly reduced in order to minimize clumping (see 43). W. F. McLimens, J. Immunol. 79, 428 (1957) as the citation 43 states at page 42, left column, lines 6-9: "Strain HeLa (Gey) was cultured in medium consisting of human, calf or horse serum, 10 %; Eagle's mixture (9) in Earle's balanced salt solution, 90 % ....". From this statement, it is presumed that the medium used in the above culture has a calcium ion concentration of at least 1.85 mM. Specifically, 10 % serum contains about 0.25 mM of a calcium ion, and since the Earle's balanced salt solution is known to contain 200 mg/liter of $CaCl_2$, 90 % of this salt solution contains about 1.6 mM of a calcium ion. As a result, the mixed medium containing 10 % serum and 90 % salt solution contains about 1.85 mM of a calcium ion.

Accordingly, although the reference states that $Ca^{++}$ should be omitted, it is apparent from the foregoing that the medium actually used contains $Ca^{++}$ in a relatively high concentration of about 1.85 mM. Thus, although the reference discloses that at this $Ca^{++}$ concentration level clumping of cells was minimized, it cannot immediately be expected from this disclosure that cells in small aggregated masses as in the present invention are grown at a high density for a long period of time. The above reference does not at all suggests that adherent cells are cultured in suspension at a high density by using a serum-free medium.

(b) Journal of Experimental Medicine, Vol. 152, 469-474 (1980)

This reference states as page 46: "HeLa-S3 cells were grown in suspension in the spinner modification of Eagle's minimum essential medium (spinner medium) supplemented with 4 % fetal calf serum. The initial cell concentration was $2 \times 10^4$ cells/ml." This statement, however, merely shows the results obtained by examining the effect of the concentration of interferon in this culture upon all surface receptors for the lectin concanavalin A. It does not at all describe the state of high density cells, particularly their clumping at low calcium ion concentration.

(c) Nature, Vol. 329, 341 (24 September 1987)

This paper describes "Transformation of cell adhesion properties by exogenously introduced E-cadherin cDNA". Table 1 at page 343 shows the results obtained by examining the $Ca^{++}$-dependent aggregation of untransformed and transformed L and F9 cells. Table 1 shows the degrees of aggregation in the presence of 1 mM $Ca^{++}$ and in the absence of $Ca^{++}$. Table 1 indicates that according to the type of the cells, the degree of aggregation may or may not depend upon the presence of $Ca^{++}$, and generally in the absence of $Ca^{++}$, aggregation does not occur. In the experiment shown in Table 1, however, the state of cell aggregation was examined only 30 minutes after the incubation, and no state of aggregation was examined after long-term cultivation. Furthermore, this paper is quite silent on the type of the medium used and the cell density. When $Ca^{++}$ is absent, the cells themselves cannot survive. This reference therefore fails to suggest the present invention.

(d) IN VITRO, 16(6), 486-490 (1980)

This reference describes "cell aggregate suspension culture for large-scale production of bio-molecules". It shows that when SV3T3 cells were cultured in Dulbecco's modified Eagle's medium containing 10 % fatal bovine serum (presumably this medium contains about 2 mM of $Ca^{++}$), and very large cell blocks were formed.

This reference, however, does not at all describe that when the culture is carried out at a high cell density in a serum-free medium as in the present invention, the aggregation of cells is adjusted by maintaining the $Ca^{++}$ concentration at a relatively low level.

The invention will now be described in greater detail.

The cells to be cultured in accordance with this invention are adherent animal cells. The adherent cells denote cells of the type which after cultivation in a serum-containing medium in a tissue culture dish, cannot be separated as living single cells by pipetting.

Suitable adherent animal cells for use in the process of this invention have the property of forming clumps containing at least 100 cells per clump on an average when they are cultured in suspension with stirring in an ordinary medium for animal cells for about 5 days. The ordinary medium contains a calcium ion in a concentration of 0.3 to 1.8 mM.

The adherent animal cells used in the process of this invention are selected from 293 strain (deposited under the ATCC No. CRL 1573) derived from human fetal kidney cells and transformants thereof which are capable of secreting a useful biologically active substance. Since in the process of this invention, cells can be cultured at a high density and the culture broth is taken out, the useful biologically active substance secreted by the cells can be separated in a high concentration from the culture broth.

Thus, according to the process of this invention, adherent animal cells secreting a useful biologically active substance can be cultured in suspension as single cells or small cell clumps in a serum-free medium, and the culturing can be carried out at a high cell density continuously for a long period of time. Hence, according to the invention, the culture broth taken out from the culture system contains the useful biologically active substance in a high concentration and its separation is economically advantageous. Accordingly, the useful biologically active substance can be industrially produced continuously and stably.

The culturing process of this invention is applied to 293 cells derived from human fetal kidney cells.

Preparation of the transformants is carried out by a general method of transfecting an expression vector containing DNA having a base sequence encoding the amino acid sequence of the desired protein into host animal cells.

Examples of the expression vector which can be transfected into the adherent animal cells include expression vectors obtained by inserting DNAs encoding useful biologically active substances exemplified below into various vectors, for example viral vectors such as BLPV, AMIV and vacccinia virus) or plasmid vectors using promotors such as SV-40 early promoter, adenovirus MLP and LRSV promoter optionally together with a selection marker.

Examples of the useful biologically active substance which are encoded by DNAs to be inserted in the expression vectors are given below.

Hormones

Erythropoetin (EPO), growth hormone, insulin, beta-endorphin, calcitonin, somatostatin, growth hormone releasing factor (GRF), caerulein, cholecystokinin, corticotropin-releasing factor, alpha-neoendorphin, pituitary gonadotropine, glucagon, LH-PH, sodium diuretic peptide, oxytocin, parathyroid hormone, secretin, THR, vasopressin, proinsulin, luteinizing hormone, enkephalin, lipocortin, somatomedin, renin, angiotensin I, angiogenic factor, thiol protease inhibitor (TPI), $\alpha_1$-antitrypsin (AAT), collagenase inhibitor, glutathione, urogastron, and hirudine.

Cytokinins

Interferon (IFN), tumor necrosis factor (TNF), interleukin 1 (IL-1), interleukin 2 (IL-2), interleukin 3 (IL-3), interleukin 4 (IL-4), B cell differentiation factor (BCDF), T cell replacing factor (TRF) 2, NKCF, leukoregulin, macrophage activating factor (MCF) and leumorphin.

Coagulation fibrolytic proteins

Protein C (PC), activated protein C (APC), tissue plasminogen activator (TPA), thrombomodulin, urokinase (UK), prourokinase (pro-UK), streptokinase, blood coagulation factor VIII, blood coagulation factor IX, blood coagulation factor XIII, and tissue factor protein S.

Enzymes

Chymosin, elastase, superoxide dismutase, A-amylase, T-4 lysozyme, alkaline phosphatase, penicillinase, T-4 ligase, beta-lactamase, beta-glucanase, beta-glucosidase, staphylokinase, lipase, penicillin G acylase, glutoamylase, pullulanase, cathechol-2,3-oygenase, prochymosin, metallothionein, oxyreductase and DNA ligase.

Vaccines

HBsAg, KEB virus antigenic protein, KATLV antigenic peptide, poliovirus antigenic protein, A-type heptatitis virus antigenic protein, HSV antigenic protein, FMDV antigenic protein, M protein of streptococcus, and diphtehric toxin antigenic proesin.

Blood constituents

Immunoglobulin, serum albumin, apolipoprotein E and apolipoprotein A-1.

Growth factor

Colony stimulation factor (CSF), epidermal growth factor (EGF), transforming growth factor (TGF), nerve growth factor (NGF), insulin-like growth factor, and macrophage proliferation factor.

Other useful biologically active substances

Protein A, beta-actin, and soybean storage protein (glycinin).

Especially preferably, the adherent animal cells are those which secrete protein C (PC) or activated protein C (APC) transfected by the above method.

The culturing method used in this invention is a method by which cells are cultured in suspension over a long period of time under controlled culture conditions while continuously or intermittently withdrawing from the culture vessel part of the spent medium with the cells contained therein or separated therefrom, and feeding a fresh medium in an amount corresponding to the amount of the medium withdrawn.

Simply stated, the culturing method of the invention may be said to be a perfusion-suspension culture method. One important point in the perfusion suspension culture is that living cells are efficiently separated from the culture broth, the spent medium is taken out of the culture vessel, and the cell living environment in the culture vessel is maintained under optimum conditions. The spent medium taken out from the culture vessel can be reused as a fresh medium by recovering useful substances contained in it or separating and removing growth inhibiting substances by means of a separating method by a membrane or a separation method by adsorption for cultivation, and adjusting the calcium ion to a predetermined level.

What is most important in the culture method of this invention is to maintain the calcium ion ($Ca^{++}$) concentration in the medium under the culturing conditions at 0.002 mM to 0.3 mM. The unit "mM" denotes the amount in millimoles of a calcium ion present in the aqueous medium (water) per liter. The calcium ion ($Ca^{++}$) means calcium metal based on a calcium compound present in a dissolved state in the medium.

The calcium ion concentration in the medium is preferably 0.02 mM to 0.25 mM, most preferably 0.05 mM to 0.20 mM. When cultured in a medium having a calcium ion concentration of more than 0.3 mM, the adherent animal cells adhere together and aggregate into many large clumps as the cultivation is continued. As the cultivation is further continued, they grow larger gradually, and the cells in the interior of the clumps die away. Furthermore, when clumps of a size above a certain limit form, the amount of a useful biologically active substance secreted per cell tends to decrease undesirably.

On the other hand, if the calcium ion concentration falls below the above-specified range, the cells no longer grow and in many cases die away. Even if the cells are growing, the amount of the useful biologically active substance secreted by the cells abruptly decreases, and the purpose of culture cannot be achieved.

Accoording to this invention, by maintaining the calcium ion concentration in the medium under the culture conditions, the cells can be cultured for a long period of time as relatively small particles even if the cell density is as high as at least $3 \times 10^6$ cells/ml, preferably at least $5 \times 10^6$ cells/ml.

Under preferred conditions, suspension cultivation at a high density of $7 \times 10^6$ to $3 \times 10^7$ cells/ml can be performed.

In a preferred embodiment of this invention, most of the cells in the culture vessel, preferably at least 90 % thereof, especially preferably at least 95 % thereof, are maintained in suspension while forming relatively small particles each containing 1.1 to 50 cells on an average, preferably 1.5 to 30 cells on an average. This can be achieved by culturing the cells in a medium having the low calcium concentration specified above and the culture system is maintained in a good suspended state by agitation and/or aeration, and efficiently supplying a fresh medium and discharging the spent medium.

The simplest method of maintaining the calcium ion concentration in the medium under cultivation conditions is to control the calcium ion concentration of a fresh medium to be fed to the culture vessel to the aforesaid range. Specifically, it is simple to use a serum-free medium having a low calcium ion concentration as the fresh medium.

The serum-free medium used in this invention will now be described.

The cultivation in accordance with this invention is carried out substantially by using a serum-free medium which does not substantially contain proteins derived from a biological source such as serum. Any basal media heretofore used for cultivation in the presence of serum may be utilized as the serum-free medium. Examples include RPMI-1640 medium, Eagle's basal medium (BME), minimum essential medium (MEM), Isocove's medium, HAM F12 medium, L-15 medium, Williams' medium, Waymouth's medium and Dulbecco's modified Eagle's medium (DME). Most of these basal media are usually sold with a calcium ion concentration of 0.3 to 1.8 mM, especially 0.5 mM to 1.5 mM, and as they are, are unsuitable for use in the low calcium ion cultivation in accordance with this invention. Accordingly, it is necessary to prepare such basal media having a calcium concentration adjusted to a low concentration, for example 0.0026 mM, and optionally add a calcium ion to adjust the total calcium ion concentration to the level preferred in this invention. It is also possible to remove part of the calcium ion from one or a mixture of commercial basal media having a relatively high calcium ion concentration and to use the resulting media as a medium with a low calcium ion concentration.

The compositions of the above basal madia intrinsically require the addition of at least 10 % of serum. In order to use them for serum-free cultivation in accordance with this invention, it is necessary to add various nutrients or growth factors or proliferation factors in place of the serum.

As required, up to 2 % by volume of serum may be added to a serum-free medium adjusted to low calcium ion concentration so long as the calcium ion concentration is confined with the range specified in this invention. For from the viewpoint of reducing cell clumping, the cultivation at a low calcium ion concentration produces sufficient effect, but from the stand-point of proliferating and maintaining the cells, the addition of some amount of serum is preferred for some types of cells. The serum may be, for example, fetal calf serum (FCS), new-born calf serum (NBCS), calf serum (CS), or horse serum (HS).

In the case of adding such serum, the calcium ion concentration should be adjusted by considering the fact that usually the serum contains some amount of a calcium ion.

Instead of utilizing the commercial serum-free media, a culture media having a low calcium ion concentration specified in this invention may be prepared by adding components usually used for cell culture, such as inorganic salts, vitamins, coenzymes, glucose, amino acids and antibiotics and further a calcium salt such as calcium chloride or calcium nitrate or both to an aqueous medium composed substantially of water, and as required adding less than 3 % by volume of serum.

Generally, cultivation of cells in suspension is started by sowing the cells at a relatively low density, for example about $5 \times 10^4$ to $1 \times 10^6$ cells/ml. At a density of up to about $3 \times 10^6$ cells/ml, the cells may be cultured by an ordinary known method, namely in a medium having a calcium ion concentration of at least 0.3 mM (serum-containing or serum-free medium), or in a low calcium ion medium in accordance with this invention. In the present invention when the cell density exceeds $3 \times 10^6$ cells/ml and the cells are in suspension, the low calcium ion medium described in this invention is used.

In the culturing process of this invention, the oxygen concentration of the culture medium is maintained constant by supplying oxygen, for example by directly supplying oxygen or an oxygen-containing gas to the suspension. As another means of supplying oxygen, an oxygen carrier, for example, may be used. The oxygen carrier is a liquid compound substantially immiscible with water and capable of dissolving oxygen. Examples are various fluorocarbons used as a material for artificial blood. When such fluorocarbons are used as means of dissolving oxygen, a fluorocarbon having dissolved oxygen therein may be added from above in the form of liquid droplets or a thin film. It is also possible to fix an oxygen-permeable Teflon or silicon tube to the inside of the culture vessel.

According to the process of this invention, adherent animal cells can be cultured in suspension continuously over a long period of time at a high cell density without the need to use an expensive microcarrier. Moreover, since this process uses a serum-free medium, it is inexpensive, and the useful biologically active substance can be easily separated from the recovered medium and purified.

In addition, since the suspension culture can be performed at a high cell density for a long period of time, the volume efficiency of the culture vessel is high and the useful biologically active substance can be produced in great quantities even in a relatively small culture vessel.

Another great advantage of this invention is that since in suspension, the cells mostly form small clumped particles each consisting of several to several tens of cells, the spent medium can be very smoothly taken out from the culture vessel. In a continuous operation of suspension culture of non-adherent cells, it is extremely difficult to take out the spent medium while avoiding inclusion of cells therein. However, no such difficulty occurs in the suspension culture in accordance with this invention.

Thus, according to this invention, the spent medium containing a relatively high concentration of the useful biologically active substance secreted by the cells can be withdrawn from the culture vessel continuously and smoothly. The process of this invention is very suitable for the industrial production of useful substances.

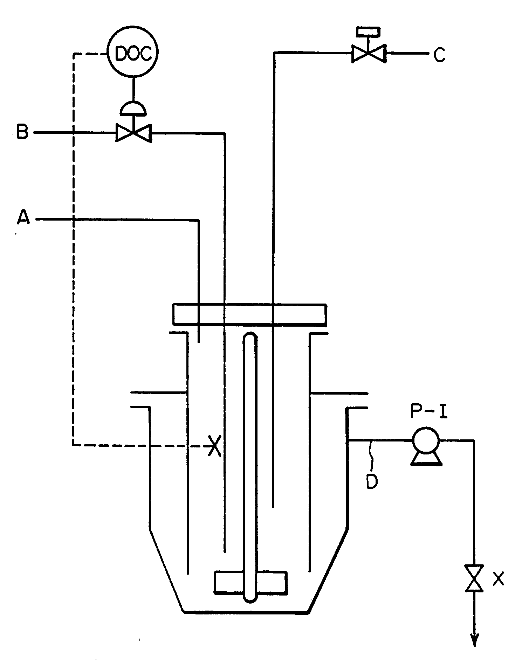

Brief Description of the Drawing

The accompanying drawing is a schematic view of the culture vessel used in the following Examples of the invention.

The following Examples illustrate the present invention.

EXAMPLE 1

1) Culture device

The culture system shown in the accompanying drawing was used. In this culture system, a settling zone partitioned by a wall inwardly of an outside wall is provided, and a discharge port for the culture medium is provided at the top. The net capacity of the culture system is about 180 ml.

2) Culture medium

The basal medium used was prepared by mixing RPMI 1640 medium, HAM F12 medium and Dulbecco modified Eagle's medium in a ratio of 2:1:1, adding glucose, amino acids, etc. (the resulting mixture is referred to as eRDF), and decreasing the calcium ion concentration of the mixture to 0.1 mM from usual 0.74 mM. Insulin, transferrin, ethanolamine and sodium selenite (to be referred to as ITES) were added as proliferation factor in a concentration of 9 $\mu$g/ml, 10 $\mu$g/ml, 10 $\mu$M, and 20 nm, respectively.

3) Method and Result of Culturing

The culture medium was fed to a net culture volume of about 180 ml into the culture vessel which had previously been autoclaved. Strain 293 originated from fetal kidney cells and obtained from ATCC were seeded in the culture vessel at a density of $0.8 \times 10^6$ cells/ml.

Oxygen gas was fed to the culture vessel through blowing nozzle (B) with automatic control so that the concentration of dissolved oxygen became 3 ppm. The culture medium in the culture vessel was maintained at 37 °C. A marine-type agitating impeller was set in the culture vessel and was rotated at a speed of 40 rpm.

The culturing was carried out batchwise for one day after the seeding, and thereafter, perfusion was started.

Pump P was operated, and the culture medium separated from the cells in the culture vessel was withdrawn from line (D), and a fresh medium in the same amount as that of the withdrawn medium was continuously fed into the culture vessel from line (A). With time, the cell density increased, and on the 6th day, reached $15 \times 10^6$ cells/ml. Thereafter, the proliferation of the cells showed a steady state. The culturing was continued for 30 days. The results are shown in Table 1.

## Table 1

### Cell: Strain 293

### Medium: ITES-eRDF (calcium ion 0.15 mM)

| Culturing time (days) | Medium replacement (ml/day) | Living cell density ($\times 10^6$ cells/ml) |
|---|---|---|
| 0 | 0 | 0.8 |
| 2 | 140 | 2.0 |
| 4 | 140 | 4.8 |
| 6 | 140 | 15 |
| 8 | 280 | 22 |
| 10 | 280 | 23 |
| 12 | 280 | 18 |
| 14 | 280 | 18 |
| 16 | 280 | 20 |
| 18 | 280 | 17 |
| 20 | 280 | 15 |
| 22 | 280 | 18 |
| 24 | 280 | 21 |
| 26 | 280 | 23 |
| 28 | 280 | 23 |
| 30 | 280 | 20 |

The average degree of aggregation in a steady state was 5 cells per particle.

COMPARATIVE EXAMPLE 1

Example 1 was repeated except that a culture medium (ITES-eRDF) having a usual calcium concentration (0.74 mM) was used instead of the culture medium used in Example 1. The results are shown in Table 2.

## Table 2

### Cell:  Strain 293
### Medium: ITES-eRDF (calcium ion 0.74 mM)

| Culturing time (days) | Medium replacement (ml/day) | Living cell density ($\times 10^6$ cells/ml) |
|:---:|:---:|:---:|
| 0 | 0 | 0.8 |
| 2 | 140 | 1.3 |
| 4 | 280 | 5.0 |
| 6 | 280 | 13 |
| 8 | 280 | 17 |
| 10 | 280 | 32 |
| 12 | 280 | 24 |
| 14 | 280 | 18 |
| 16 | 280 | 18 |
| 18 | 280 | 10 |
| 20 | 280 | 8 |
| 22 | 280 | 5 |
| 24 | 280 | 3 |
| 26 | 280 | 1.3 |
| 28 | 280 | 0.5 |
| 30 | 280 | 0.2 |

The average degree of aggregation in a steady state was $7 \times 10^3$ cells per particle.

EXAMPLE 2

1) Culture device

The culture system shown in the acompanying drawing was used. In this culture system, a settling zone partitioned by a wall inwardly of an outside wall is provided, and a discharge port for the culture medium is provided at the top. The net capacity of the culture system is about 180 ml.

2) Culture medium

The basal medium used was prepared by mixing RPMI 1640 medium, HAM F12 medium and Dulbecco modified Eagle's medium in a ratio of 2:1:1, adding glucose, amino acid, etc. (the resulting mixture is referred to as eRDF), and removing calcium salts (calcium chloride, calcium nitrate) other than calcium panthotenate (the resulting medium is referred to as low Ca eRDF). Insulin, transferrin, ethanolamine and sodium selenite IITES) was added as proliferation factors in a concentration of 9 $\mu$g/ml, 10 $\mu$g/ml, 10 $\mu$M and 20 nM, respectively.

3) Method and Result of Culturing

The culture medium was fed to a net culture volume of about 180 ml into the culture vessel which had previously been autoclaved. Protein C-producing strain 293 #3, which had been obtained by introducing a DNA fragment encoding the amino acid sequence of human protein C in accordance with the method described in Japanese Laid-Open Patent Publication No. 111690/1987 into strain 293 originated from human fetal kidney cells procured from ATCC, was seeded in the culture medium.

Oxygen gas was fed into the culture vessel through blowing nozzle (B) with automatic control so that the concentration of dissolved oxygen became 3 ppm.

The culture medium in the vessel was maintained at 37 °C. A marine-type agitation impeller was set in the culture vessel, and rotated at a speed of 40 rpm.

The culturing was carried out batchwise for one day after the seeding, and then perfusion was started. Pump P was operated, and the medium separated from the cells in the culture vessel was withdrawn from line (D), and a fresh medium in the same amount as that of the withdrawn medium was continuously fed from line (A).

The results and the number of medium replacements are shown in Table 3.

### Table 3

#### Cells: 293 #3 strain

#### Medium: ITES + low Ca eRDF (Ca$^{++}$ was only 0.0026 mM from calcium panthotenate)

| Culturing time (day) | Medium replacement (ml/day) | Living cell density (x $10^6$ cells/ml) | PC | |
|---|---|---|---|---|
| | | | ($\mu$g/ml) | (mg/day) |
| 0 | 0 | 1.0 | – | – |
| 2 | 140 | 2.4 | 0.26 | 0.04 |
| 3 | 140 | 2.7 | 0.31 | 0.04 |
| 4 | 140 | 4.7 | 0.88 | 0.12 |
| 5 | 280 | 5.6 | 1.4 | 0.39 |
| 6 | 280 | 8.4 | 1.5 | 0.42 |
| 7 | 280 | 14 | 1.1 | 0.31 |

The average degree of aggregation in a steady state was 7 cells per particle.

COMPARATIVE EXAMPLE 2

Example 2 was repeated except that usual eRDF (Ca$^{++}$ concentration 0.74) was used instead of the eRDF used in Comparative Example 2.

The results are shown in Table 4.

## Table 4

Cells: 293 #3 strain

Medium: ITES + eRDF (with a usual calcium ion concentration of 0.74 mM)

| Culturing time (day) | Medium re-placement (ml/day) | Living cell density (x $10^6$ cells/ml) | PC | |
|---|---|---|---|---|
| | | | ($\mu$g/ml) | (mg/day) |
| 0 | 0 | 1.0 | – | – |
| 1 | 140 | 0.9 | 0.82 | 0.11 |
| 3 | 140 | 3.6 | 2.2 | 0.31 |
| 5 | 280 | 4.0 | 2.2 | 0.62 |
| 7 | 280 | 4.7 | 2.2 | 0.62 |
| 11 | 280 | 5.8 | 1.9 | 0.53 |
| 13 | 280 | 4.6 | 1.1 | 0.31 |
| 15 | 280 | 4.0 | 0.66 | 0.18 |
| 18 | 280 | 3.7 | 0.56 | 0.16 |
| 20 | 280 | 3.7 | 0.22 | 0.06 |

The average degree of aggregation in a steady state was $2 \times 10^3$ cells per particle.

EXAMPLE 3

Example 2 was repeated except that 0.01 mM calcium chloride was further added to the culture medium.

The results are shown in Table 5.

## Table 5

Cells: $293$ #3 strain

Medium:  0.01 mM $CaCl_2$ + ITES + low Ca eRDF

| Culturing time (day) | Medium re-placement (ml/day) | Living cell density ($\times 10^6$ cells/ml) | PC | |
|---|---|---|---|---|
| | | | ($\mu$g/ml) | (mg/day) |
| 0 | 0 | 0.9 | – | – |
| 2 | 140 | 2.1 | 1.2 | 0.17 |
| 3 | 140 | 3.3 | 1.4 | 0.20 |
| 4 | 140 | 5.8 | 2.1 | 0.29 |
| 5 | 280 | 8.3 | 2.3 | 0.64 |
| 6 | 280 | 17 | 2.7 | 0.76 |
| 7 | 280 | 21 | 2.4 | 0.67 |

The average degree of aggregation in a steady state was 8 cells per particle.

EXAMPLE 4

Example 2 was repeated except that 0.04 mM calcium chloride was further added to the culture medium.

The results are shown in Table 6.

## Table 6

Cells: 293 #3

Culture medium: 0.04 mM $CaCl_2$ + ITES + low Ca
eRDF

| Culturing time (day) | Medium re- placement (ml/day) | Living cell density (x $10^6$ cells/ml) | PC | |
|---|---|---|---|---|
| | | | ($\mu$g/ml) | (mg/day) |
| 0 | 0 | 0.9 | – | – |
| 2 | 140 | 2.1 | 1.8 | 0.25 |
| 3 | 140 | 2.8 | 2.1 | 0.29 |
| 4 | 140 | 5.4 | 4.7 | 0.66 |
| 5 | 280 | 8.7 | 4.5 | 1.3 |
| 6 | 280 | 14 | 3.3 | 0.92 |
| 7 | 280 | 19 | 3.4 | 0.95 |

The average degree of aggregation in a steady state was 7 cells per particle.

14

EXAMPLE 5

Example 2 was repeated except that 0.1 mM calcium chloride was added to the culture medium. The results are shown in Table 7.

## Table 7

### Cells: 293 #3

### Medium: 0.1 mM $CaCl_2$ + ITES + low Ca eRDF

| Culturing time (day) | Medium replacement (ml/day) | Living cell density ($x\ 10^6$ cells/ml) | PC | |
|---|---|---|---|---|
| | | | ($\mu$g/ml) | (mg/day) |
| 0 | 0 | 0.9 | – | – |
| 2 | 140 | 1.5 | 1.0 | 0.14 |
| 3 | 140 | 2.1 | – | – |
| 4 | 140 | – | 1.6 | 0.22 |
| 6 | 280 | 8.0 | 3.6 | 1.0 |
| 7 | 280 | 12 | 5.8 | 1.6 |
| 9 | 420 | 14 | 4.1 | 1.7 |
| 10 | 420 | 12 | 4.2 | 1.8 |
| 12 | 420 | 11 | 3.9 | 1.6 |
| 14 | 420 | 14 | 4.0 | 1.7 |
| 17 | 420 | 18 | 3.2 | 1.3 |
| 20 | 420 | 19 | 3.0 | 1.3 |
| 22 | 420 | 15 | 3.1 | 1.3 |
| 23 | 420 | 14 | 4.1 | 1.7 |
| 27 | 420 | 12 | 2.7 | 1.1 |
| 29 | 420 | 13 | 2.2 | 0.92 |

The average degree of aggregation in a steady state was 6 cells per particle.

COMPARATIVE EXAMPLE 3

Example 2 was repeated except that protein C-producing strain BHK 229-10 obtained by introducing a DNA fragment encoding the amino acid sequence of human protein C into BHK strain procured from ATCC was used as the cells to be cultured; up to the 13th day, the culturing was carried out by using the medium used in Example 3; and that on the 14th day and onwards, the cultivation was carried out by using the medium used in Comparative Example 2.

The results are shown in Table 8.

## Table 8

### Cells: BHK 229-10

Medium: (A) 0.01 mM $CaCl_2$ + ITES + low Ca
eRDF

(B) ITES + eRDF (usual calcium ion
concentration 0.74 mM)

| Culturing time (day) | Medium | Medium replacement (ml/day) | Living cell density (x $10^6$ cells/ml) | PC | |
|---|---|---|---|---|---|
| | | | | (μg/ml) | (mg/day) |
| 0 | A | 0 | 1.0 | – | – |
| 1 | A | 80 | 1.2 | – | – |
| 2 | A | 240 | 1.5 | – | – |
| 4 | A | 240 | 2.2 | 0.23 | 0.22 |
| 6 | A | 240 | 2.9 | 0.33 | 0.08 |
| 7 | A | 430 | 3.4 | 0.21 | 0.09 |
| 8 | A | 430 | 4.9 | 0.44 | 0.19 |
| 9 | A | 430 | 7.0 | – | – |
| 10 | A | 430 | 8.0 | 0.27 | 0.12 |
| 13 | A | 560 | 7.4 | 0.26 | 0.15 |
| 14 | B | 560 | about 7.0 | 2.1 | 1.2 |
| 15 | B | 560 | about 7.0 | 3.3 | 1.8 |
| 19 | B | 560 | about 7.0 | 2.9 | 1.6 |
| 21 | B | 560 | about 7.0 | 2.5 | 1.4 |
| 23 | B | 560 | about 7.0 | 2.2 | 1.2 |

The average degree of aggregation in a steady state was 40 cells per particle.

Table 8 shows that when BHK cells were cultivated in a low calcium medium for a certain period of time, and then in a medium having a usual calcium ion concentration, the cell clumps did not become large, and the cells could be cultured in suspension.

COMPARATIVE EXAMPLE 4

Comparative Example 3 was repeated except that the culture medium used in Comparative Example 1 was used throughout the cultivation period.

The results are shown in Table 9.

## Table 9

### Cells: BHK 229-10 strain
### Medium: ITES + eRDF (usual calcium ion
### concentration 0.74 mM)

| Culturing time (day) | Medium replacement (ml/day) | Living cell density (x $10^6$ cells/ml) | PC | |
|---|---|---|---|---|
| | | | ($\mu$g/ml) | (mg/day) |
| 0 | 0 | 1.0 | – | – |
| 2 | 140 | 0.9 | 3.2 | 0.45 |
| 8 | 140 | 0.3 | 1.0 | 0.14 |
| 14 | 140 | 0.2 | 1.0 | 0.14 |
| 22 | 140 | 0.5 | 1.6 | 0.22 |
| 30 | 140 | 0.6 | 1.2 | 0.17 |

The average degree of aggregation in a steady state was $3 \times 10^4$ cells per particle.

COMPARATIVE EXAMPLE 5

Example 5 was repeated except that protein C-producing CHO Z4-I5 strain obtained by introducing a DNA fragment encoding the amino acid sequence of human protein C into CHO strain were used instead of the cells cultured in Example 5.

17

The results are shown in Table 10.

## Table 10

### Cells: CHO Z4-I5 strain

### Medium: 0.1 mM CaCl$_2$ + ITES + low Ca eRDF

| Culturing time (day) | Medium replacement (ml/day) | Living cell density (x 10$^6$ cells/ml) | PC | |
|---|---|---|---|---|
| | | | ($\mu$g/ml) | (mg/day) |
| 0 | 0 | 0.5 | – | – |
| 3 | 140 | 1.5 | 5.2 | 0.73 |
| 4 | 140 | 2.1 | 5.5 | 0.77 |
| 6 | 140 | 2.7 | 6.0 | 0.84 |
| 7 | 280 | 5.0 | 4.9 | 1.4 |
| 10 | 430 | 7.0 | 4.9 | 2.1 |
| 12 | 560 | 9.9 | 6.2 | 3.5 |

The average degree of aggregation in a steady state was 30 cells per particle.

## Claims

1. A process for continuously culturing adherent animal cells selected from 293 strain (ATCC No. CRL 1573) derived from human fetal kidney cells and transformants thereof which are capable of secreting a useful biologically active substance in a serum-free medium, characterized in that
   (1) a fresh medium is fed into a culture vessel, and a spent medium is withdrawn from the vessel,
   (2) the adherent animal cells in the serum-free medium in the vessel are maintained in suspension,
   (3) the adherent animal cells in suspension are caused to exist at a density of at least 3 x 10$^6$ cells/ml, and
   (4) the concentration of a calcium ion in the medium under the culturing conditions is maintained at 0.002 mM to 0.3 mM.

2. The process of claim 1 in which the concentration of a calcium ion in the medium under the culturing conditions is maintained at 0.002 mM to 0.25 mM.

3. The process of claim 1 or 2 in which most of the adherent animal cells in suspension are present as aggregated particles each containing 1.5 to 50 cells on an average.

4. The process of claim 1 or 2 in which most of the adherent animal cells in suspension are present as aggregated particles each containing 1.5 to 30 cells on an average.

5. The process of any one of claims 1 to 4 in which the adherent animal cells in suspension are present at a density of at least 5 x 10$^6$ cells/ml.

6. The process of any one of claims 1 to 5 in which the feeding of the fresh medium, and the withdrawal of the spent medium, into and from the culture vessel are carried out continuously or intermittently, and the liquid level in the culture vessel is maintained substantially constant.

18

7. The process of any one of claims 1 to 6 in which the suspended state of the adherent animal cells in the culture vessel is maintained by agitation and/or aeration.

**Patentansprüche**

1. Verfahren zur kontinuierlichen Züchtung von adherenten tierischen Zellen, ausgewählt aus dem Stamm 293 (ATCC Nr. CRL 1573), abgeleitet von menschlichen fetalen Nierenzellen und Transformanten davon, die eine nützliche biologisch aktive Substanz in einem serumfreien Medium ausscheiden können, dadurch **gekennzeichnet**, daß

(1) ein frisches Medium in ein Kulturgefäß eingespeist wird und ein verbrauchtes Medium dem Gefäß entnommen wird,

(2) die adherenten tierischen Zellen in dem serum-freien Medium in dem Gefäß in Suspension gehalten werden,

(3) die adherenten tierischen Zellen in Suspension bei einer Dichte von mindestens $3 \times 10^6$ Zellen/ml gezüchtet werden, und

(4) die Konzentration eines Calciumions in dem Medium unter den Kulturbedingungen bei 0,002 mM bis 0,3 mM gehalten wird.

2. Verfahren nach Anspruch 1, dadurch **gekennzeichnet**, daß die Konzentration eines Calciumions in dem Medium unter den Kulturbedingungen bei 0,002 mM bis 0,25 mM gehalten wird.

3. Verfahren nach Anspruch 1 oder 2, dadurch **gekennzeichnet**, daß die meisten der adherenten tierischen Zellen in Suspension als aggregierte Teilchen vorhanden sind, die jeweils 1,5 bis 50 Zellen im Durchschnitt enthalten.

4. Verfahren nach Anspruch 1 oder 2, dadurch **gekennzeichnet**, daß die meisten der adherenten tierischen Zellen in Suspension als aggregierte Teilchen vorhanden sind, die jeweils 1,5 bis 30 Zellen im Durchschnitt enthalten.

5. Verfahren nach einem der Ansprüche 1 bis 4, dadurch **gekennzeichnet**, daß die adherenten tierischen Zellen in Suspension in einer Dichte von mindestens $5 \times 10^6$ Zellen/ml vorhanden sind.

6. Verfahren nach einem der Ansprüche 1 bis 5, dadurch **gekennzeichnet**, daß das Einspeisen des frischen Mediums und das Abziehen des verbrauchten Mediums in und aus dem Kulturgefäß kontinuierlich oder intermittierend durchgeführt werden und der Flüssigkeitsspiegel in dem Kulturgefäß im wesentlichen konstant gehalten wird.

7. Verfahren nach einem der Ansprüche 1 bis 6, dadurch **gekennzeichnet**, daß der suspendierte Zustand der adherenten tierischen Zellen in dem Kulturgefäß durch Rühren und/oder Belüften aufrechterhalten wird.

**Revendications**

1. Procédé de culture en continu de cellules animales adhérentes choisies parmi la souche 293 (ATCC N° CRL 1573) dérivée de cellules de rein foetal humain et ses transformants qui sont capables de sécréter une substance utile biologiquement active dans un milieu exempt de sérum, caractérisé en ce que

(1) on introduit un milieu frais dans un récipient de culture, et on soutire un milieu épuisé du récipient,

(2) on maintient en suspension les cellules animales adhérentes dans le milieu exempt de sérum dans le récipient,

(3) on fait de sorte que les cellules animales adhérentes en suspension existent à une densité d'au moins $3 \times 10^6$ cellules/ml, et

(4) on maintient la concentration d'un ion calcium dans le milieu dans les conditions de culture à une valeur de 0,002 mM à 0,3 mM.

2. Procédé selon la revendication 1, dans lequel la concentration en ions calcium dans le milieu dans les conditions de culture est maintenue à une valeur de 0,002 mM à 0,25 mM.

3.  Procédé selon la revendication 1 ou 2, dans lequel la plupart des cellules animales adhérentes en suspension sont présentes sous forme de particules agrégées contenant chacune 1,5 à 50 cellules en moyenne.

4.  Procédé selon la revendication 1 ou 2, dans lequel la plupart des cellules animales adhérentes en suspension sont présentes sous forme de particules agrégées contenant chacune 1,5 à 30 cellules en moyenne.

5.  Procédé selon l'une quelconque des revendications 1 à 4, dans lequel les cellules animales adhérentessuspension sont présentes à une densité d'au moins $5 \times 10^6$ cellules/ml.

6.  Procédé selon l'une quelconque des revendications 1 à 5, dans lequel l'introduction du milieu frais, et le soutirage du milieu épuisé, dans et à partir du récipient de culture sont réalisés en continu ou par intermittence, et le niveau de liquide dans le récipient de culture est maintenu sensiblement constant.

7.  Procédé selon l'une quelconque des revendications 1 à 6, dans lequel l'état en suspension des cellules animales adhérentes dans le récipient de culture est maintenu par agitation et/ou aération.